# EUROPÄISCHE PATENTANMELDUNG

(11) **EP 1 177 770 A2**
(43) Veröffentlichungstag der Anmeldung: **06.02.2002**
(21) Anmeldenummer: 01000334.1
(22) Anmeldetag: 01.08.2001
(51) Int. Cl.: A61B 17/22

(54) **Elektroden-Anordnung für eine Stosswellenquelle**

(30) Priorität: 03.08.2000 DE 10037790
(71) Anmelder: Philips Corporate Intellectual Property GmbH, 52064 Aachen (DE); Koninklijke Philips Electronics N.V., 5621 BA Eindhoven (NL)
(72) Erfinder: Haaks, Wilfried Dr., c/o Philips Corp. Intell., 52064 Aachen (DE); Schwieker, Horst Hartwig, c/o Phil. Corp. Intell., 52064 Aachen (DE)
(74) Vertreter: Volmer, Georg, Dipl.-Ing.

(57) **Zusammenfassung**

Es wird eine Elektroden-Anordnung zur Erzeugung von Stoßwellen durch elektrische Entladung zwischen Elektrodenspitzen (12, 13) beschrieben, die insbesondere als ESWL (Extrakorporale-Stoßwellen-Lithotripsie)-Elektrode in eine Stoßwellenquelle zur Nierensteinzertrümmerung oder als Elektrode in einem Gerät zur extrakorporalen Stoßwellentherapie (ESWT) geeignet ist. Die Elektroden-Anordnung zeichnet sich insbesondere dadurch aus, dass mindestens eine der Elektrodenspitzen (12, 13) in einen zugeordneten Elektrodenhalter (14,15) auswechselbar einsetzbar ist, wobei die Elektrodenspitze und der Elektrodenhalter zur gegenseitigen Arretierung korrespondierende Passkonturen aufweisen. Dies ermöglicht einen sehr kostengünstigen Austausch der abgebrannten Elektrodenspitzen durch den Anwender.

## Beschreibung

Die Erfindung betrifft eine Elektroden-Anordnung zur Erzeugung von Stoßwellen durch elektrische Entladung zwischen Elektrodenspitzen, die insbesondere als ESWL (Extrakorporale-Stoßwellen-Lithotripsie) -Elektrode in der Stoßwellenquelle eines Lithotripters zur Nierensteinzertrümmerung oder als Elektrode in einem Gerät zur extrakorporalen Stoßwellentherapie (ESWT) geeignet ist.

Elektroden-Anordnungen dieser Art dienen zum Beispiel in der Lithotripsie zur Unterwasser-Stoßwellenerzeugung, bei der eine Funkenstrecke zwischen den Elektrodenspitzen in einem ersten Brennpunkt eines ellipsenähnlichen Reflektors liegt, dessen zweiter Brennpunkt auf das Gebiet ausgerichtet wind, in dem ein Konkrement, wie zum Beispiel ein Nierenstein eines Patienten, zu zertrümmern ist.

Die Elektrodenspitzen unterliegen dabei einem relativ starken Abbrand, so dass die ESWL- oder ESWT-Elektrode im allgemeinen nach der Behandlung von einem bis drei Patienten ausgetauscht werden muss. Dies wird insbesondere aus Kostengründen als nachteilig angesehen.

Aus der DE 38 14 468 ist eine "Elektrode zur Zertrümmerung von Nierensteinen" bekannt, bei der eine der Elektrodenspitzen an einen Dorn gelötet ist, der auswechselbar in eine Hülse eingesetzt wird, während die andere Elektrodenspitze in einer entsprechenden Öffnung eines Käfigs ebenfalls durch Löten befestigt ist. Dadurch soll die Elektrode durch Einlöten neuer Elektrodenspitzen wiederverwendbar gemacht werden.

Ein wesentlicher Nachteil hierbei besteht jedoch darin, dass die Elektrode zu diesem Zweck zum. Hersteller geschickt werden muss, so dass für den Anwender allenfalls eine geringe Kosteneinsparung im Vergleich zu einer Neuanschaffung entsteht, der Wartungsaufwand aber ansonsten der gleiche ist, wie bei den Elektroden, die nicht wiederverwendet werden können.
Eine wesentliche, der Erfindung zugrunde liegende Aufgabe besteht deshalb darin, eine Elektroden-Anordnung der eingangs genannten Art zu schaffen, deren Betrieb wesentlich geringere Kosten und einen geringeren Wartungsaufwand verursacht.

Gelöst wird diese Aufgabe mit einer Elektroden-Anordnung der eingangs genannten Art, die sich dadurch auszeichnet, dass mindestens eine der Elektrodenspitzen in einen zugeordneten Elektrodenhalter auswechselbar einsetzbar ist, wobei die Elektrodenspitze und der Elektrodenhalter zur gegenseitigen Arretierung korrespondierende Passkonturen aufweisen.

Ein wesentlicher Vorteil dieser Lösung besteht darin, dass die Elektrodenspitzen in einfacher Weise durch den Anwender selbst ausgetauscht werden können. Dies ist nicht nur effektiver als ein kompletter Austausch der Elektrode, sondern es werden auch die Kosten erheblich vermindert.

Die Unteransprüche haben vorteilhafte Weiterbildungen der Erfindung zum Inhalt.

Die Ausführung gemäß Anspruch 2 sowie deren Weiterbildung gemäß Anspruch 3 haben den Vorteil, dass die Elektrodenspitze einfach herstellbar ist und an einem bekannten Elektrodenhalter nur geringfügige konstruktive Änderungen vorgenommen werden müssen.

Wenn im Gegensatz dazu die Ausführung gemäß den Ansprüchen 4 oder 5 gewählt wird, ist keine Vorspanneinrichtung erforderlich, so dass die Montage in bestimmten Fällen noch etwas einfacher, die Herstellung dafür etwas aufwendiger sein könnte.

Die erfindungsgemäße Elektroden-Anordnung ist schließlich in besonderer Weise als Stoßwellenelektrode oder auch ESWL-Elektrode zur Anwendung in einer Stoßwellenquelle zur Nierensteinzertrümmerung oder eines anderen Konkrementes geeignet.

Weitere Einzelheiten, Merkmale und Vorteile der Erfindung ergeben sich aus der folgenden Beschreibung einer bevorzugten Ausführungsform anhand der Zeichnung.

Es zeigt:
Fig. 1 eine schematische Darstellung eines Lithotripters;
Fig. 2 eine schematische Darstellung einer Elektroden-Anordnung;
Fig. 3 einen schematischen Querschnitt durch die in Figur 2 gezeigte Elektroden-Anordnung; und
Fig. 4 eine vergößerte Darstellung einer Elektrodenspitze.

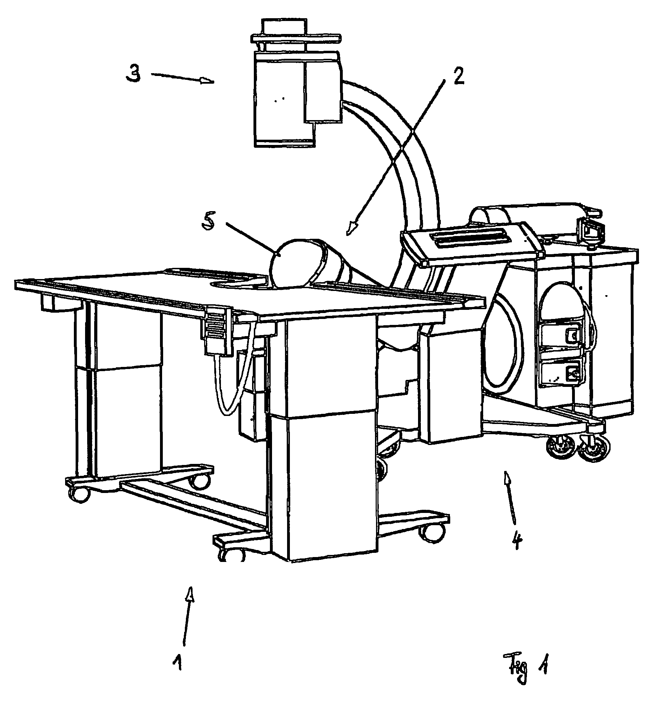

Ein typischer Behandlungsplatz für die Lithotripsie, d.h. ein Lithotripter, umfasst gemäß Figur 1 einen Patiententisch 1, eine Stoßwellenquelle 2, ein Röntgengerät 3 sowie verschiedene Steuer- und Bedieneinheiten 4. Die Stoßwellenquelle weist bekanntlich eine mit Wasser gefüllte Gummiblase 5 auf, die mit einem auf dem Patiententisch 1 liegenden Patienten in Berührung gebracht wind, um einen möglichst störungsfreie Übergang der erzeugten Stoßwellen in den Körper des Patienten zu erzielen.

Die Stoßwellen werden durch die Funkenstrecke einer Elektroden-Anordnung (auch als Stosswellenelektrode oder ESWL-Elektrode bezeichnet) erzeugt, die sich innerhalb der Gummiblase, und zwar in einem ersten Brennpunkt eines ellipsenähnlichen Reflektors befindet. Die Gestaltung und Anordnung wird dabei in bekannter Weise so vorgenommen, dass der zweite Brennpunkt innerhalb des Körpers des Patienten dort liegt, wo ein Nierenstein oder ein anderes Konkrement zu zerstören ist. Mit Hilfe des Röntgengerätes 3 kann die Wirksamkeit der Behandlung beobachtet werden.

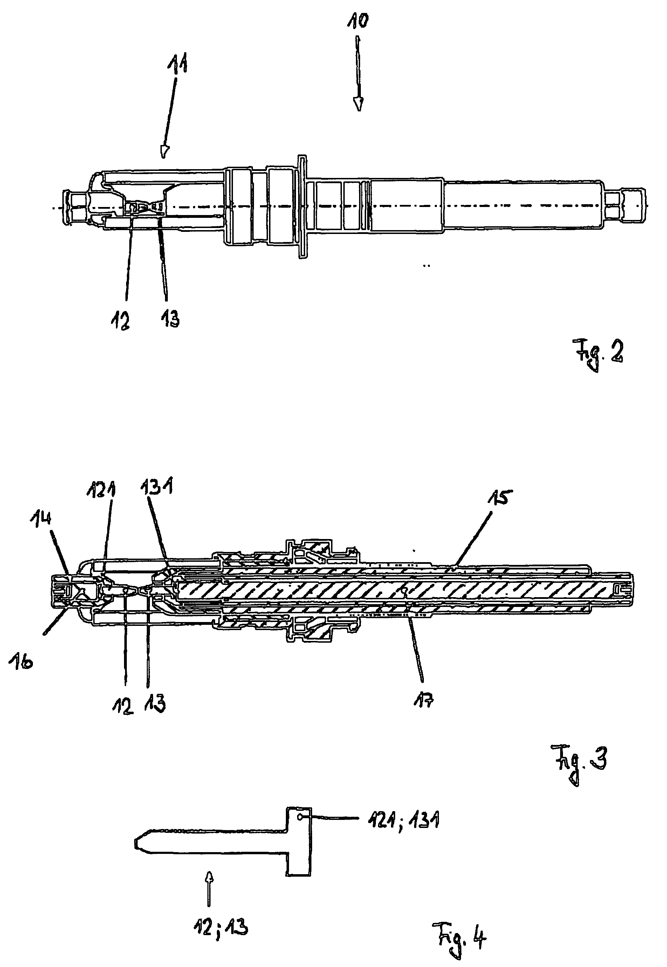

Figur 2 zeigt eine einzelne Elektroden-Anordnung mit einem Gehäuse 10 von außen. Das Gehäuse 10 umfasst in bekannter Weise Verriegelungsmittel zum Einsetzen der Elektrode in eine Stoßwellenquelle, sowie Kontakte zur Zuführung einer elektrischen Spannung. An einer Seite befindet sich ein Käfig 11, in dem eine erste und eine zweite Elektrodenspitze 12, 13 eine Funkenentladungsstrecke abgrenzen.

Figur 3 zeigt den inneren Aufbau dieser Elektroden-Anordnung und die Form der Elektrodenspitzen 12, 13. Die Elektrodenspitzen weisen an ihren der Funkentladungsstrecke abgewandten Enden jeweils eine sockelähnliche Umfangserweiterung auf, die einen Vorsprung 121; 131 bildet.
Die erste Elektrodenspitze 12 befindet sich in einem ersten Elektrodenhalter 14, während für die zweite Elektrodenspitze 13 ein zweiter Elektrodenhalter 15 vorgesehen ist. Beide Elektrodenhalter sind hohl und an ihren der Funkenentladungsstrecke abgewandten (ersten) Enden offen, so dass eine Elektrodenspitze in dieses erste Ende eingesetzt und bis zu dem zweiten gegenüberliegenden Ende der Elektrodenhalter 14, 15 hindurchgeführt werden kann.

An ihren zweiten Enden weisen die Elektrodenhalter 14, 15 jeweils eine Bohrung mit einer solchen Ausnehmung (Innenkontur) auf, dass die Elektrodenspitzen 12; 13 bis zu ihrem eine korrespondierende Außenkontur aufweisenden Vorsprung 121; 131 durch diese Bohrungen in den Funkenentladungsraum geführt werden können.

Durch die korrespondierenden Passkonturen an der Elektrodenspitze bzw. dem zweiten Ende der Elektrodenhalter ist eine einfache und genaue Positionierung der freien Enden der Elektrodenspitzen in dem Funkenentladungsraum möglich.

Die Elektrodenspitzen werden in ihrer Position entweder dadurch befestigt, dass die Passung der Konturen eine Presspassung ist, oder sie werden wie in dem in Figur 3 gezeigten Fall durch jeweils eine Vorspanneinrichtung in Form einer ersten und zweiten Druckschraube 16; 17 arretiert, die in Axialrichtung von dem ersten Ende des jeweiligen Elektrodenhalters in diesen eingeführt und durch Eingriff mit einem Gewinde an dessen Innenwandung gegen die Elektrodenspitze geschraubt wird. Dadurch wird gleichzeitig auch eine elektrische Verbindung zwischen den Elektrodenspitzen 12, 13 und dem betreffenden Elektrodenhalter 14, 15 bzw. einem dort vorgesehenen elektrischen Anschluß hergestellt.

Figur 4 zeigt schließlich eine der Elektrodenspitzen 12; 13 in vergrößerter Darstellung, in der insbesondere der Vorsprung 121; 131 deutlich wird.

Wenn nach der Behandlung von etwa einem bis drei Patienten die Elektrodenspitzen so weit abgebrannt sind, dass eine Funkenentladung nicht mehr stattfindet, so löst und entfernt der Anwender die beiden Druckschrauben 16, 17 und entnimmt die Elektrodenspitzen aus den Elektrodenhaltern 14, 15. Nach dem Einstecken neuer Elektrodenspitzen 12, 13 werden die Druckschrauben wieder eingesetzt und angezogen, so dass der Betrieb fortgesetzt werden kann.

Als Alternative zu den beschriebenen Druckschrauben können die Elektrodenspitzen auch selbst ein Gewinde aufweisen, mit dem sie mit einem korrespondierenden Gewinde in den Elektrodenhaltern verschraubt und auf diese Weise arretiert werden.

Eine weitere Möglichkeit besteht darin, einen Bajonett-Verschluss zwischen den Elektrodenspitzen und den Elektrodenhaltern vorzusehen, der die Arretierung bewirkt.

Bei oben erläuterten Ausführungen werden die Elektrodenspitzen jeweils von innen durch die Elektrodenhalter geführt und arretiert. Umgekehrt ist es natürlich auch möglich, die Arretierung so zu gestalten, dass die Elektrodenspitzen von außen, das heißt von der Seite der Funkenentladungsstrecke in die Elektrodenhalter eingesteckt und - sei es durch ein Gewinde oder einen Bajonett-Verschluss - an diesen befestigt werden.

## Patentansprüche

1. Elektroden-Anordnung zur Erzeugung von Stoßwellen durch elektrische Entladung zwischen Elektrodenspitzen,
**dadurch gekennzeichnet, dass** mindestens eine der Elektrodenspitzen (12, 13) in einen zugeordneten Elektrodenhalter (14, 15) auswechselbar einsetzbar ist, wobei die Elektrodenspitze und der Elektrodenhalter zur gegenseitigen Arretierung korrespondierende Passkonturen aufweisen.

2. Elektroden-Anordnung nach Anspruch 1,
**dadurch gekennzeichnet, dass** die Passkontur durch mindestens einen sich im wesentlichen radial erstreckenden Vorsprung (121; 131) an der Elektrodenspitze sowie eine dazu korrespondierende Ausnehmung an dem Elektrodenhalter (14; 15) gebildet ist, gegen die die Elektrodenspitze (12; 13) mit dem Vorsprung mittels einer Vorspanneinrichtung (16; 17) gedrückt wird.

3. Elektroden-Anordnung nach Anspruch 2,
**dadurch gekennzeichnet, dass** die Vorspanneinrichtung durch eine in dem Elektrodenhalter (14; 15) gegen die Elektrodenspitze (12; 13) schraubbare Druckschraube (16; 17) gebildet ist, mit der auch eine elektrische Verbindung zwischen der Elektrodenspitze und dem Elektrodenhalter hergestellt wird.

4. Elektroden-Anordnung nach Anspruch 1,
**dadurch gekennzeichnet, dass** die Passkontur durch korrespondierende Gewinde an dem Elektrodenhalter (14; 15) und der Elektrodenspitze (12; 13) gebildet ist.

5. Elektroden-Anordnung nach Anspruch 1,
**dadurch gekennzeichnet, dass** die Passkontur durch einen Bajonettverschluß zwischen Elektrodenhalter (14; 15) und Elektrodenspitze (12; 13) gebildet ist.

6. Stoßwellenelektrode (ESWL-Elektrode) mit einer Elektroden-Anordnung nach einem der vorhergehenden Ansprüche.

7. Elektrodenspitze zum austauschbaren Einsetzen in eine Elektroden-Anordnung nach einem der Ansprüche 1 bis 5.

8. Stoßwellenquelle mit einer Elektroden-Anordnung nach Anspruch 1.

9. Lithotripter mit einer Stoßwellenquelle nach Anspruch 8.
